(19) **Europäisches Patentamt / European Patent Office / Office européen des brevets**

(11) **EP 1 886 688 A1**

(12) # EUROPEAN PATENT APPLICATION
published in accordance with Art. 153(4) EPC

(43) Date of publication:
**13.02.2008 Bulletin 2008/07**

(21) Application number: **06756692.7**

(22) Date of filing: **29.05.2006**

(51) Int Cl.:
*A61K 31/7088* [(2006.01)]  *A61K 31/7105* [(2006.01)]
*A61K 31/711* [(2006.01)]  *A61K 31/713* [(2006.01)]
*A61K 47/16* [(2006.01)]  *A61K 47/24* [(2006.01)]
*A61K 48/00* [(2006.01)]  *A61P 35/00* [(2006.01)]

(86) International application number:
**PCT/JP2006/310647**

(87) International publication number:
**WO 2006/129594 (07.12.2006 Gazette 2006/49)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.05.2005 JP 2005156622**

(71) Applicant: **Nippon Shinyaku Co., Ltd. Kyoto-shi, Kyoto 601-8550 (JP)**

(72) Inventors:
• **FUKUI, Yoshiharu**
  **5250055 (JP)**
• **SAHEKI, Akira**
  **6100354 (JP)**

(74) Representative: **Pautex Schneider, Nicole Véronique et al**
  **Novagraaf International SA**
  **25, avenue du Pailly**
  **1220 Les Avanchets - Geneva (CH)**

(54) **METHOD FOR PRODUCING NUCLEIC ACID-CONTAINING COMPLEX PREPARATION**

(57)    A main object of the present invention is to provide a method of preparing a nucleic-acid-containing complex formulation which can be sterilized by filtration and administered intravenously to human, and can retain stability of polynucleotides included in the nucleic-acid-containing complex formulation.

The present invention relates to a method of preparing a nucleic-acid-containing complex formulation, comprising the following steps:

mixing a solution comprising two separate single-stranded polynucleotides (for example, poly I and poly C) capable of forming a double strand and a solution comprising a cationic carrier or the ingredients thereof to form the cationic carrier, and

performing a dispersion treatment to the mixture.

EP 1 886 688 A1

**Description**

FIELD OF THE INVENTION

The present invention relates to a method of preparing a nucleic-acid-containing complex formulation comprising a complex of polynucleotides with a cationic carrier.

BACKGROUND ART

In recent years, it has been studied to apply polynucleotides such as short interfering RNA (hereinafter, referred to as "siRNA") and poly I:C to medicine (see, for example, Patent documents 1 to 3). However, polynucleotides cannot effectively exhibit its biological activity in a cell due to an extremely low intracellular uptake. For the effective exhibition of the biological activity, there is a method of forming a complex of polynucleotides with a cationic carrier and administrating the complex.

A conventional method of preparing the complex includes a method of mixing a double-stranded polynucleotide with a cationic carrier. However, the complex prepared by the method is generally very large in particle size ranging from several micrometers to several hundred micrometers in diameter. Therefore, the nucleic-acid-containing complex formulation comprising the complex cannot be sterilized by filtration. In addition, the formulation also has a problem that embolization or the like may occur in capillaries when being intravenously administered to human.

As a method to solve the problem, there has been published a preparation method which is characterized in that two separate nucleic acid polymers in the form of a single strand are separately added to a cationic carrier, and then the dispersion treatment is performed (see, for example, Patent document 4).

Meanwhile, it goes without saying that polynucleotides as an active ingredient are desired to be more stable because they are susceptible to degradation by a nuclease or heat.

[Patent document 1] WO 99/20283 pamphlet
[Patent document 2] WO 99/48531 pamphlet
[Patent document 3] WO 2004/106511 pamphlet
[Patent document 4] WO 99/61032 pamphlet

DISCLOSURE OF THE INVENTION

PROBLEM TO BE SOLVED BY THE INVENTION

A main obj ect of the present invention is to provide a method of preparing a nucleic-acid-containing complex formulation which can be sterilized by filtration and administered intravenously to human, and can retain stability of polynucleotides.

MEANS TO SOLVE THE PROBLEM

The inventors of the present invention have conducted extensive researches to find that in a preparation of a nucleic-acid-containing complex formulation using two complementary polynucleotides and a cationic carrier, the above object is achieved by dissolving two separate single-stranded polynucleotides in one solution, adding the solution to the cationic carrier, and mixing and dispersing the two polynucleotides with the cationic carrier, thereby accomplishing the present invention.

The present invention can include, for example, the following (1) and (2).

(1) A method of preparing a nucleic-acid-containing complex formulation, comprising the following steps:

mixing a solution comprising two separate single-stranded polynucleotides capable of forming a double strand and a solution comprising a cationic carrier or the ingredients thereof to form the cationic carrier, and performing a dispersion treatment to the mixture.

(2) A nucleic-acid-containing complex formulation obtained by the method of the above (1).

BEST MODE FOR CARRYING OUT THE INVENTION

Polynucleotides according to the present invention can include polydeoxyribonucleotides and polyribonucleotides. In addition, polydeoxyribonucleotides can include oligodeoxyribonucleotides, and polyribonucleotides can include oligoribonucleotides.

A part of a polydeoxyribonucleotide may be substituted with a ribonucleotide. A part of a polyribonucleotide may be substituted with a deoxyribonucleotide.

The polynucleotides can include, for example, polyinosinic acid (hereinafter, referred to as "poly I"), polycytidylic acid (hereinafter, referred to as "poly C"), polyadenylic acid, polyuridylic acid, poly(cytidylic acid, uridylic acid) poly (cytidylic acid, 4-thio uridylic acid), polycytidylic acid /poly-L-lysine, poly(1-vinyl cytidylic acid), an antisense DNA, an antisense RNA, a DNA enzyme, a ribozyme, a decoy, an aptamer, and a sense strand or an antisense strand of a double-stranded oligonucleotide which can exert RNA interference (hereinafter, referred to as "RNAi") In addition, the double-stranded oligonucleotide which can exert RNAi can include siRNA, micro RNA (miRNA), short hairpin RNA (shRNA) and small nuclear RNA (snRNA) . Meanwhile, the term "RNAi" is familiar to persons skilled in the art. It is a phenomenon that a mRNA having a sequence complementary to a sense strand or an antisense strand of the introduced double-stranded oligonucleotide is specifically degraded so that synthesis of the gene product coded by the mRNA is inhibited.

The base number of polynucleotides is not particularly limited, but is suitably 5000 or less, preferably 2000 or less. The base number can be appropriately selected depending on the natures of polynucleotides. Meanwhile, in the present invention, the base numbers of oligodeoxyribonucleotides and oligoribonucleotides are each in the range of 2 to 99. The term "base number" means a base number calculated from a peak of chromatogram of a molecular weight standard and that of a sample in a so-called size exclusion chromatography. That is to say, it represents the base number calculated from maximum frequency of the chromatogram.

When the polynucleotides according to the present invention are poly I and poly C, the base numbers each may suitably be in the range of 30 to 2000, preferably in the range of 60 to 1000, more preferably in the range of 100 to 500.

When the polynucleotides according to the present invention are a sense strand and an antisense strand of a double-stranded oligonucleotide which can exert RNAi, the base numbers each may suitably be in the range of 10 to 50, preferably in the range of 12 to 40, more preferably in the range of 15 to 30.

The whole or a part the polynucleotides may be chemically modified. The chemically modified polynucleotides can include, for example, a poly I chemically modified in part such as poly(7-deazainosinic acid) and poly(2'-azidoinosinic acid); and a poly C chemically modified in part such as poly (5-bromocytidylic acid), poly(2-thiocytidylic acid) and poly (cytidine-5'-thiophosphoric acid). In addition, in order to enhance in vivo stability such as nuclease resistance, the polynucleotides may be modified at least partially in a glycocomponent or a phosphate backbone constituting the polynucleotides.

The polynucleotides can be prepared by a method known to persons skilled in the art. For example, the polynucleotides can be prepared in a solid phase synthetic method or a liquid phase synthetic method using a phosphoroamidite or a triester using an automatic synthesizer for nucleic acid or manually. In addition, the polynucleotides can also be prepared by dissociating the double strand of a double-stranded polynucleotide by non-enzymatic treatment with heating at 60 °C or more, or enzymatic treatment using helicase or the like.

The two polynucleotides according to the present invention are not particularly limited so long as they are capable of forming a double strand, and may be a combination of a polydeoxynucleotide and a polyribonucleotide. In addition, the base numbers of the two polynucleotides are not necessarily the same.

The term "capable of forming a double strand" means having a complementary sequence to such a degree that they can form double strand when the two polynucleotides are mixed under physiological conditions. The above term "under physiological conditions" means a condition controlled to a pH, a salt composition and a temperature, which are similar to those of in vivo conditions. For example, the conditions can include a condition where pH is in the range of 5 to 8 and sodium chloride concentration is 150 mM at room temperature.

The cationic carrier according to the present invention is not particularly limited so long as it is a pharmaceutically acceptable cationic carrier. The carrier can include a cationic carrier comprising 2-O-(2-diethylaminoethyl) carbamoyl-1,3-O-dioleoyl glycerol (hereinafter, referred to as "Compound A") and a phospholipid as a essential components (hereinafter, referred to as "Carrier A"), a cationic carrier comprising N-[1-(2,3-dioleyloxy)propyl]-N,N,N-trimethylammonium chloride and a phospholipid as a essential components, OligofectAMINE (registered trademark) (manufactured by Invitrogen), Lipofectin (registered trademark) (manufactured by Invitrogen), Lipofectamine (registered trademark) (manufactured byInvitrogen), Lipofectamine2000 (registered trademark) (manufactured by Invitrogen), DMRIE-C (registered trademark) (manufactured by Invitrogen), GeneSilencer (registered trademark) (manufactured by Gene Therapy Systems), TransMessenger(registered trademark) (manufactured by Qiagen) and TransITTKO (registered trademark) (manufactured by Mirus). Among them, Carrier A is particularly preferable.

The method of preparing a cationic carrier according to the present invention is described below referring to the Carrier A as an example.

Compound A constituting Carrier A can be synthesized by a method disclosed in WO 94/19314 pamphlet.

The phospholipid constituting Carrier A is not particularly limited so long as it is a pharmaceutically acceptable phospholipid. It can include phosphatidylcholine such as egg yolk phosphatidylcholine, phosphatidylethanolamine, phosphatidylinositol, phosphatidylserine, sphingomyelin; and lecithin such as egg yolk lecithin and soybean lecithin. Among them, lecithin is particularly preferable.

Carrier A can be prepared by mixing Compound A with a phospholipid, and then performing a dispersion treatment to the mixture in a conventional method. In the dispersion treatment, an emulsifying dispersion machine or the like can appropriately be used. The emulsifying dispersion machine is not particularly limited so long as it is a machine for producing pharmaceutical products. The machine can include an ultrasonic dispersingmachine such as SONIFIER (registered trademark) (manufactured by BRANSON, hereinafter, the same is applied); and a high-pressurized emulsifying dispersion machine such as Microfluidizer (registered trademark) (manufactured by Microfluidics, hereinafter, the same is applied), Nanomizer (registered trademark) (manufactured by YOSHIDA KIKAI, hereinafter, the same is applied), DeBEE2000 (registered trademark) (manufactured by BEE international, hereinafter,thesameisapplied),Ultimizer(registered trademark) (manufactured by SUGINO MACHINE, hereinafter, the same is applied) and Nano2000 (registered trademark) (manufactured by NIHON BEE, hereinafter, the same is applied).

The solution used for dispersing Compound A and a phospholipid is not particularly limited so long as it is a pharmaceutically acceptable solution. The solution can include water for injection, distilled water for injection, an electrolytic solution such as saline; and a carbohydrate solution such as glucose injection and maltose injection.

In the dispersion treatment, processing conditions, processing time and processing temperatures are selected appropriately.

The composition ratio of Compound A and a phospholipid used in preparing Carrier A (a weight of a phospholipid / a weight of Compound A) may vary depending on the kind of a phospholipid used, natures of two polynucleotides mixed with Carrier A in preparing a nucleic-acid-containing complex formulation and so on, and the ratio may suitably be in the range of 0.1 to 10, preferably in the range of 0.5 to 5, and more preferably in the range of 1 to 2.

The nucleic-acid-containing complex formulation according to the present invention (hereinafter, referred to as "the formulation of the present invention") can be prepared by allowing two polynucleotides to dissolve in one solution, mixing the solution with a solution in which a cationic carrier is dispersed or a solution comprising the ingredients thereof to form the cationic carrier, and performing a dispersion treatment to the mixture in a conventional method. In the dispersion treatment, an emulsifying dispersion machine or the like can suitably be used. The emulsifying dispersion machine is not particularly limited so long as it is a machine using pharmaceutical uses. The emulsifying dispersion machine can include an ultrasonic dispersing machine such as SONIFIER (registered trademark); and a high-pressurized emulsifying dispersion machine such as Microfluidizer (registered trademark), Nanomizer (registered trademark), DeBEE2000 (registered trademark), Ultimizer (registered trademark) and Nano2000 (registered trademark).

The solution used for dissolving polynucleotides is not particularly limited so long as it is a pharmaceutically acceptable solution. The solution can include water for injection, distilled water for injection, an electrolytic solution such as saline; and a carbohydrate solution such as glucose injection and maltose injection.

In the dispersion treatment, processing conditions, processing time and processing temperatures are selected appropriately.

The composition ratio of polynucleotides and a cationic carrier used in preparing the formulation of the present invention (a total weight of polynucleotides / a weight of a cationic carrier) may vary depending on natures of the polynucleotides used and the cationic carrier used and so on. The ratio may suitably be in the range of 0.0005 to 1, and preferably in the range of 0.001 to 0.4. In addition, when the cationic carrier is Carrier A, the ratio may suitably be in the range of 0.0005 to 1, preferably in the range of 0.001 to 0.4, and more preferably in the range of 0.01 to 0.2.

In the formulation of the present invention, pharmaceutically acceptable additives can be blended in addition to two polynucleotides and a cationic carrier. The additives can include emulsifying auxiliary agents (for example, fatty acids such as caprylic acid, capric acid, lauric acid, myristic acid, palmitic acid, stearic acid, oleic acid, linoleic acid, arachidonic acid or docosahexaenoic acid, and their pharmaceutically acceptable salts, albumin, and dextran), stabilizers (for example, cholesterol, and phosphatidic acid), isotonisities (for example, sodium chloride, glucose, maltose, lactose, sucrose, and trehalose), pH adjustment agents (for example, hydrochloric acid, nitric acid, phosphoric acid, acetic acid, sodium hydroxide, potassium hydroxide, triethanolamine). These can be used alone or as a mixture of two or more thereof. The contents of the additives are suitably 90 % by weight or less, preferably 70 % by weight or less, and more preferably 50 % by weight or less in the formulation of the present invention.

The additives can be added at an appropriate time of the process either before or after a dispersion treatment.

After a dispersion treatment, the formulation of the present invention can optionally be sterilized by filtration with a 0.2 $\mu$m membrane filter.

The formulation of the present invention can be processed into a lyophilized formulation. The lyophilized formulation can be prepared by freeze-drying the liquid formulation of the present invention in a conventional method. For example, after sterilizing the liquid formulation of the present invention adequately, the formulation is dispensed into vial containers each in a predetermined amount, and then the formulation is subjected to preliminary freezing at about -40 to -20 °C for about 2 hours, followed by a first drying at about 0 to 10 °C under a reduced pressure and a secondary drying at about 15 to 25 °C under a reduced pressure. Thereafter, in general, the vials are filled with nitrogen gas and sealed with stoppers to give the lyophilized formulation of the present invention.

In general, the lyophilized formulation of the present invention can be reconstituted in an optional suitable solution (reconstituting solution) and be used. The reconstituting solution can include water for injection, saline, and the other standard infusions. The amount of the reconstituting solution may vary depending on an usage and the like, and is not particularly limited, but the appropriate amount may be 0.5 to 5 times the amount of the original liquid formulation before the freeze-drying or not more than 500 mL.

The administration route of the formulation of the present invention is not particularly limited so long as it is a pharmaceutically acceptable administration route. The route can include intravenous administration, intraarterial administration, oral administration, intra-tissue administration, transdermal administration, mucosal administration and intrarectal administration. Among them, intravenous administration, transdermal administration and mucosal administration are preferred. In addition, it can be administrated as a suitable pharmaceutical form such as various types of injections, an oral agent, a drop, an inhalant, an ocular instillation, an ointment, a lotion and a suppository depending on the administration route.

EXAMPLE

The present invention is illustratedmore specificallybelow by referring to the following Examples, Comparative Example and Test Examples. However, the present invention is not limited thereto.

During the filter sterilization with a 0.2μm membrane filter in Examples 1 to 7 and Comparative Example 1, clogging of filters did not happen. The yields of filtrates were in the range of 98 to 102 % for all samples, indicating that sterilization efficiency of approximately 100 % was attainable.

Example 1

Two hundred fifteen mL of water for injection (manufactured by OTSUKA PHARMACEUTICAL, hereafter the same is applied) was added to 3 g of CompoundA, 5 g of purified egg yolk lecithin (manufactured by Q.P. Corporation, hereafter the same is applied) and 50 g of maltose (manufactured by HAYASHIBARA, hereafter the same is applied) with stirring. The mixture was subjected to a dispersion treatment for 30 minutes using a homogenizer to obtain a crude dispersion solution containing a cationic carrier. The crude dispersion solution was further subjected to a dispersion treatment under a processing pressure at 108 MPa for 30 minutes using a pressurized emulsifying dispersion machine [Microfluidizer (registered trademark) (hereinafter, the same is applied)] to obtain a dispersion solution containing 8 g of a cationic carrier. Then, 250 mg of poly I with a base number of about 170 and 250 mg of poly C with a base number of about 180 were dissolved in 200 mL of water for injection. Two hundred mL of this solution was added to the whole volume of the dispersion solution containing a cationic carrier with stirring. The mixture was furthermore subjected to a dispersion treatment under a processing pressure at 108 MPa for 2 hours using a pressurized emulsifying dispersion machine, followed by sterilization by filtration with a 0.2 μm membrane filter to obtain the formulation of the present invention.

Subsequently, 2 mL of the formulation of the present invention was dispensed into each vial and processed into a lyophilized formulation according to a conventional method. The obtained lyophilized formulation was reconstituted by an addition of 1.8 mL of water for injection. The average diameter of complex particles in the reconstituted formulation of the present invention was determined to be 152 nm by means of the particle diameter measuring apparatus [Nicomp370 (registered trademark) manufactured by Particle Sizing Systems Inc., hereafter the same is applied] employing a dynamic light scattering method. Besides, no particle with a diameter of 1 μm or more was included.

Example 2

Two hundred twenty one mL of water for injection was added to 1.5 g of Compound A, 2.5 g of purified egg yolk lecithin and 50 g of maltose with stirring. The mixture was subjected to a dispersion treatment for 30 minutes using a homogenizer to obtain a crude dispersion solution containing a cationic carrier. The crude dispersion solution was further subjected to a dispersion treatment under a processing pressure at 108 MPa for 30 minutes using a pressurized emulsifying dispersion machine to obtain a dispersion solution containing 4 g of a cationic carrier. Then, 125 mg of poly I with a base number of about 400 and 125 mg of poly C with a base number of about 380 were dissolved in 200 mL of water for injection. Two hundred mL of this solution was added to the whole volume of the dispersion solution containing a cationic carrier with stirring. The mixture was furthermore subjected to a dispersion treatment under a processing pressure at 108 MPa for 2 hours using a pressurized emulsifying dispersion machine, followed by sterilization by filtration with a 0.2 μm membrane filter to obtain the formulation of the present invention.

Subsequently, 1 mL of the formulation of the present invention was dispensed into each vial and processed into a lyophilized formulation according to a conventional method. The obtained lyophilized formulation was reconstituted by an addition of 5 mL of water for injection. The average diameter of complex particles in the reconstituted formulation of the present invention was determined to be 146 nm by means of the particle diameter measuring apparatus employing a dynamic light scattering method. Besides, no particle with a diameter of 1 μm or more was included.

Example 3

Two hundred fifteen mL of water for injection was added to 3 g of Compound A, 5 g of palmitoyl-oleyl-phosphatidylcholine (manufactured by NOF Corporation) and 50 g of maltose with stirring. The mixture was subjected to a dispersion treatment for 30 minutes using a homogenizer to obtain a crude dispersion solution containing a cationic carrier. The crude dispersion solution was further subjected to a dispersion treatment under a processing pressure at 108 MPa for 30 minutes using a pressurized emulsifying dispersion machine to obtain a dispersion solution containing 8 g of a cationic carrier. Then, 250 mg of poly I with a base number of about 310 and 250 mg of poly C with a base number of about 330 were dissolved in 200 mL of water for injection. Two hundred mL of this solution was added to the whole volume of the dispersion solution containing a cationic carrier with stirring. The mixture was furthermore subjected to a dispersion treatment under a processing pressure at 108 MPa for 2 hours using a pressurized emulsifying dispersion machine, followed by sterilization by filtration with a 0.2 μm membrane filter to obtain the formulation of the present invention.

Subsequently, 2 mL of the formulation of the present invention was dispensed into each vial and processed into a lyophilized formulation according to a conventional method. The obtained lyophilized formulation was reconstituted by an addition of 1.8 mL of water for injection. The average diameter of complex particles in the reconstituted formulation of the present invention was determined to be 142 nm by means of the particle diameter measuring apparatus employing a dynamic light scattering method. Besides, no particle with a diameter of 1 μm or more was included.

Example 4

A volume of water for injection was added to 0.12 g of Compound A, 0.2 g of purified egg yolk lecithin and 2 g of maltose to give the final volume 10 mL, and then stirred thoroughly to obtain a crude dispersion solution containing a cationic carrier. The crude dispersion solution was further subjected to a dispersion treatment using an ultrasonic dispersion machine with an output of 100 W for 15 minutes to obtain a dispersion solution containing 0.32 g of a cationic carrier. Then, 10 mg of poly I with a base number of about 310 and 10 mg of poly C with a base number of about 330 were dissolved in 50 mL of water for injection. Five mL of this solution was added to 5 mL of the dispersion solution containing a cationic carrier with stirring. The mixture was furthermore subj ected to a dispersion treatment using an ultrasonic dispersion machine [SONIFIER (registered trademark) (hereinafter, the same is applied)] with an output of 100 W for 15 minutes, followed by sterilization by filtration with a 0.2 μm membrane filter to obtain the formulation of the present invention.

Subsequently, 1 mL of the formulation of the present invention was dispensed into each vial and processed into a lyophilized formulation according to a conventional method. The obtained lyophilized formulation was reconstituted by an addition of 0. 87 mL of water for injection. The average diameter of complex particles in the reconstituted formulation of the present invention was determined to be 145 nm by means of the particle diameter measuring apparatus employing a dynamic light scattering method. Besides, no particle with a diameter of 1 μm or more was included.

Example 5

A volume of water for injection was added to 0.12 g of Compound A, 0.2 g of purified egg yolk lecithin and 2 g of maltose to give the final volume of 10 mL, and then stirred thoroughly to obtain a crude dispersion solution containing a cationic carrier. The crude dispersion solution was further subjected to a dispersion treatment using an ultrasonic dispersion machine with an output of 100 W for 15 minutes to obtain a dispersion solution containing 0.32 g of a cationic carrier. Then, 30 mg of poly I with a base number of about 310 and 30 mg of poly C with a base number of about 330 were dissolved in 10 mL of water for injection. Five mL of this solution was added to 5 mL of the dispersion solution containing a cationic carrier with stirring. The mixture was furthermore subjected to a dispersion treatment using an ultrasonic dispersion machine with an output of 100 W for 15 minutes, followed by sterilization by filtration with a 0.2 μm membrane filter to obtain the formulation of the present invention.

Subsequently, 1 mL of the formulation of the present invention was dispensed into each vial and processed into a lyophilized formulation according to a conventional method. The obtained lyophilized formulation was reconstituted by an addition of 0.87 mL of water for injection. The average diameter of complex particles in the reconstituted formulation of the present invention was determined to be 163 nm by means of the particle diameter measuring apparatus employing a dynamic light scattering method. Besides, no particle with a diameter of 1 μm or more was included.

Example 6

A volume of water for injection was added to 0.16 g of N-[1-(2,3-dioleyloxy)propyl]-N,N,N-trimethylammonium chloride (manufactured by TOKYO CHEMICAL INDUSTRY), 0.16 g of dioleoyl phosphatidyl ethanolamine (manufactured by NOF Corporation) and 2 g of maltose to give the final volume of 10 mL, and then stirred thoroughly to obtain a crude dispersion solution containing a cationic carrier. The crude dispersion solution was further subjected to a dispersion treatment using an ultrasonic dispersion machine with an output of 100 W for 15 minutes to obtain a dispersion solution containing 0.32 g of a cationic carrier. Then, 10 mg of poly I with a base number of about 310 and 10 mg of poly C with a base number of about 330 were dissolved in 10 mL of water for injection. Five mL of this solution was added to 5 mL of the dispersion solution containing a cationic carrier with stirring. The mixture was furthermore subjected to a dispersion treatment using an ultrasonic dispersion machine with an output of 100 W for 15 minutes, followed by sterilization by filtration with a 0.2 $\mu$m membrane filter to obtain the formulation of the present invention.

Subsequently, 1 mL of the formulation of the present invention was dispensed into each vial and processed into a lyophilized formulation according to a conventional method. The obtained lyophilized formulation was reconstituted by an addition of 0.87 mL of water for injection. The average diameter of complex particles in the reconstituted formulation of the present invention was determined to be 151 nm by means of the particle diameter measuring apparatus employing a dynamic light scattering method. Besides, no particle with a diameter of 1 $\mu$m or more was included.

Example 7

A volume of water for injection was added to 0 . 6 g of Compound A, 1 g of purified egg yolk lecithin and 10 g of maltose to give the final volume of 50 mL, and then stirred thoroughly to obtain a crude dispersion solution containing a cationic carrier. The crude dispersion solution was further subjected to a dispersion treatment under a processing pressure at 135 MPa for 90 minutes using a pressurized emulsifying dispersion machine to obtain a dispersion solution containing 1.6 g of a cationic carrier. Then, 25 mg each of two mutually complementary oligoribonucleotides both having a base number of 21 were dissolved in 25 mL of a maltose solution in water for injection containing 5 g of maltose. Twenty-five mL of this solution was added to 25 mL of the dispersion solution containing a cationic carrier with stirring. The mixture was furthermore subjected to a dispersion treatment under a processing pressure at 135 MPa for 40 hours using a pressurized emulsifying dispersion machine, followed by sterilization by filtration with a 0.2 $\mu$m membrane filter to obtain the formulation of the present invention.

Subsequently, 1 mL of the formulation of the present invention was dispensed into each vial and processed into a lyophilized formulation according to a conventional method. The obtained lyophilized formulation was reconstituted by an addition of 0.87 mL of water for injection. The average diameter of complex particles in the reconstituted formulation of the present invention was determined to be 147 nm by means of the particle diameter measuring apparatus employing a dynamic light scattering method. Besides, no particle with a diameter of 1 $\mu$m or more was included.

Comparative Example 1

Two hundred fifteen mL of water for injection was added to 3 g of Compound A, 5 g of purified egg yolk lecithin and 50 g of maltose with stirring. The mixture was subjected to a dispersion treatment for 30 minutes using a homogenizer to obtain a crude dispersion solution containing a cationic carrier. The crude dispersion solution was further subjected to a dispersion treatment under a processing pressure at 108 MPa for 30 minutes using a pressurized emulsifying dispersion machine to obtain a dispersion solution containing 8 g of a cationic carrier. One hundred mL of a solution including 250 mg of poly I with a base number of about 180 and 100 mL of a solution including 250 mg of poly C with a base number of about 200 was simultaneously added to the whole volume of the dispersion solution containing a cationic carrier with stirring. The mixture was subjected to a dispersion treatment under a processingpressure at 108 MPa for 2 hours using a pressurized emulsifying dispersion machine, followed by sterilization by filtration with a 0.2 $\mu$m membrane filter to obtain the comparative formulation.

Subsequently, 2 mL of the comparative formulation was dispensed into each vial and processed into a lyophilized formulation according to a conventional method. The obtained lyophilized formulation was reconstituted by an addition of 1.8 mL of water for injection. The average diameter of complex particles in the reconstituted comparative formulation was determined to be 149 nm by means of the particle diameter measuring apparatus employing a dynamic light scattering method. Besides, no particle with a diameter of 1 $\mu$m or more was included.

Test Example 1

Thermal stability of poly I and poly C included in the formulation of the present invention obtained in Example 1 and the comparative formulation obtained in Comparative example 1 was evaluated from the change of each base number.

In the experiment, each formulation was stored for 2 weeks under the conditions of 40 °C and 75 % relative humidity, and was defatted with diethyl ether (manufactured by NACALAI TESQUE, hereafter the same is applied). Subsequently, each formulation was subjected to a gel filtration chromatography which is a kind of size exclusion chromatography using TSK-Gel G5000PW$_{XL}$ column (manufactured by Tosoh, hereafter the same is applied). The absorbances at 250 nm and 260 nm were measured, and then the base number of each of poly I and poly C was calculated.

The result is shown in Table 1.

As shown in Table 1, the base numbers of poly I and poly C included in the comparative formulation obtained in Comparative Example 1 decreased during the storage. On the other hand, the base numbers of poly I and poly C included in the formulation of the present invention obtained in Example 1 did not change before and after the storage. The formulation of the present invention was more stable to heat than the comparative formulation.

[TABLE 1]

| | | before storage | after storage |
|---|---|---|---|
| Example 1 | poly I | 167 ± 1 | 169 ± 3 |
| | poly C | 174 ± 3 | 176 ± 4 |
| Comparative Example 1 | poly I | 182 ± 5 | 136 ± 11 |
| | poly C | 203 ± 1 | 150 ± 18 |

Test Example 2

Thermal stability of poly I and poly C included in the formulation of the present invention obtained in Examples 2 and 3 was evaluated from the change of each base number.

In the experiment, each formulation was stored for 6 months under the conditions of 40 °C and 75 % relative humidity, and was defatted with diethyl ether. Subsequently, each formulation was subjected to a gel filtration chromatography which is a kind of size exclusion chromatography using TSK-Gel G500OPW$_{XL}$ column. The absorbances at 250 nm and 260 nm were measured, and then base number of each of poly I and poly C was calculated.

The result is shown in Table 2.

As shown in Table 2, the base numbers of poly I and poly C included in the formulation of the present invention obtained in Examples 2 and 3 did not change before and after the storage. The formulations of the present invention were stable to heat.

[TABLE 2]

| | | before storage | after storage |
|---|---|---|---|
| Example 2 | poly I | 400 ± 1 | 397 ± 8 |
| | poly C | 381 ± 2 | 376 ± 3 |
| Example 3 | poly I | 309 ± 1 | 305 ± 5 |
| | poly C | 328 ± 0 | 324 ± 3 |

Test Example 3

Thermal stability of oligoribonucleotide included in the formulation of the present invention obtained in Example 7 was evaluated from the change of the base number of the oligoribonucleotide.

In the experiment, each formulation was stored for 6 months under the conditions of 40 °C and 75 % relative humidity, and was defatted with diethyl ether. Subsequently, the base numbers of the oligoribonucleotide were measured by anion exchange chromatography with DNAPac PA-100 column (manufactured by DIONEX) .

The result is shown in Table 3.

As shown in Table 3, the base numbers of oligoribonucleotide included in the formulation of the present invention obtained in Example 7 did not change before and after the storage. The formulation of the present invention was stable to heat.

[TABLE 3]

|  | before storage | after storage |
|---|---|---|
| Example 7 | $21 \pm 0$ | $21 \pm 0$ |

Test Example 4

A change of pharmacological activity of the formulation of the present invention obtained in Example 2, which was stored for 6 months under the conditions of 40 °C and 75 % relative humidity, was evaluated from inhibitory effect on the growth of A431 cells: human epidermoid carcinoma.

(1) Method of experiment

A suspension of A431 cells adjusted to a density of $10^5$ cells/mL was dispensed into eachwell of 96 well plates at 90 μL/well. A431 cells were allowed to adhere to the plate by being left for 4 to 6 hours. Subsequently, the formulations of the present invention having various concentrations were added. After three days, color reaction with MTT method was conducted and the absorbance at 570 nm was measured for each well with a microplate reader. Cells of a negative control were cultured in the same way mentioned above, except that saline was added instead of the formulation of the present invention.

(2) Evaluation

The evaluation was made by estimating $IC_{50}$ (ng/mL), based on cell growth inhibition rate (%) calculated by the following formula. $IC_{50}$ here means the total concentrations of poly I and poly C.

$$\text{Cell growth inhibition rate (\%)} = (1-[\text{an absorbance of a cell treated with a medicine / an absorbance of a cell of negative control}]) \times 100$$

(3) Test result

The result is shown in Table 4.
As shown in Table 4, $IC_{50}$ of the formulation of the present invention obtained in Example 2 did not change before and after the storage. That shows the pharmacological activity was maintained.

[TABLE 4]

|  | before storage | after storage |
|---|---|---|
| Example 2 | $7.6 \pm 1.2$ | $8.1 \pm 1.5$ |

Test Example 5

The existence form of two polynucleotides in a nucleic-acid-containing complex formulation were evaluated by measuring a distance between the two polynucleotides through Fluorescence Resonance Energy Transfer (hereinafter, referred to as "FRET") method.
The FRET method is a method familiar to persons skilled in the art. Specifically, this measures relative positions between fluorescent molecules of an energy donor and an energy acceptor as a fluorescent signal excited by light absorption.

(1) Preparation of test sample

(i) Preparations of fluorescently labeled poly I and poly C

A fluorescently labeled poly I was prepared by introducing Alexa Fluor568 (manufactured by Molecular Probe) into poly I with a base number of about 170 according to a method known to persons skilled in the art. Similarly, a fluorescently labeled poly C was prepared by introducing Alexa Fluor546 (manufactured by Molecular Probe) into poly C with a base number of about 180.

(ii) Preparations of the formulation of the present invention and a control formulation

The formulation of the present invention containing both the fluorescently labeled poly I and the fluorescently labeled poly C obtained in the above (i), and a control formulation containing only the fluorescently labeled poly C were prepared under the same conditions as in Example 1.

(iii) Preparations of the comparative formulation and a control formulation

The comparative formulation containing both the fluorescently labeled poly I and the fluorescently labeled poly C obtained in the above (i), and a control formulation containing only the fluorescently labeled poly C were prepared under the same conditions as in Comparative Example 1.

(iv) Preparations of a positive control solution and a control solution

The positive control solution was prepared by dissolving both the fluorescently labeled poly I and the fluorescently labeled poly C obtained in the above (i) in physiological saline, and a control solution was prepared by dissolving only the fluorescently labeled poly C in physiological saline.

(2) Evaluation

The evaluation was made by calculating FRET efficiency from fluorescence intensities of the formulation of the present invention, the comparative formulation, the positive control solution and their respective control prepared in the above (1). FRET efficiency means a value which is obtained by subtracting a value, which is obtained by dividing a fluorescence intensity of a test sample containing both the fluorescently labeled poly I and the fluorescently labeled poly C by that of the control sample containing only the fluorescently labeled poly C, from 1.

The higher the FRET efficiency is, the nearer the distance between poly I and poly C in a test sample is judged to be.

(3) Test result

The test result is shown in Table 5.

As shown in Table 5, the distance between poly I and poly C in the formulation of the present invention was shorter than that in the comparative formulation, and equivalent to that in the positive control. Namely, Table 5 shows that the distance between poly I and poly C in the formulation of the present invention was equal to that in a double-stranded poly I and poly C.

[TABLE 5]

|  | FRET efficiency |
| --- | --- |
| The formulation of the present invention | $0.59 \pm 0.00$ |
| Comparative formulation | $0.20 \pm 0.07$ |
| Positive control | $0.61 \pm 0.03$ |

**Claims**

**1.** A method of preparing a nucleic-acid-containing complex formulation, comprising the following steps:

mixing a solution comprising two separate single-stranded polynucleotides capable of forming a double strand and a solution comprising a cationic carrier or the ingredients thereof to form the cationic carrier, and performing a dispersion treatment to the mixture.

2. The method of preparing the nucleic-acid-containing complex formulation according to claim 1, wherein the polynucleotide is a polydeoxyribonucleotide or a polyribonucleotide.

3. The method of preparing the nucleic-acid-containing complex formulation according to claim 1, wherein the polynucleotide is an oligodeoxyribonucleotide or an oligoribonucleotide.

4. The method of preparing the nucleic-acid-containing complex formulation according to claim 1, wherein the polynucleotide is polyinosinic acid, polycytidylic acid, polyadenylic acid, polyuridylic acid, poly(cytidylic acid, uridylic acid), poly(cytidylic acid, 4-thio uridylic acid), polycytidylic acid poly-L-lysine, poly(1-vinyl cytidylic acid), an antisense DNA, an antisense RNA, a DNA enzyme, a ribozyme, a decoy, an aptamer, or a sense strand or an anti-sense strand of a double-stranded oligonucleotide which can exert RNA interference.

5. The method of preparing the nucleic-acid-containing complex formulation according to claim 1, wherein the polynucleotide is polyinosinic acid, polycytidylic acid, or a sense strand or an anti-sense strand of a double-stranded oligonucleotide which can exert RNA interference.

6. The method of preparing the nucleic-acid-containing complex formulation according to any one of claims 1 to 5, wherein the cationic carrier is a cationic carrier comprising 2-O-(2-diethylamincethyl)carbamoyl-1,3-O-dioleoyl glycerol and a phospholipid as essential components.

7. The method of preparing the nucleic-acid-containing complex formulation according to claim 6, wherein the phospholipid is lecithin.

8. A nucleic-acid-containing complex formulation obtained by the method according to any one of claims 1 to 7.

## INTERNATIONAL SEARCH REPORT

| | International application No. |
|---|---|
| | PCT/JP2006/310647 |

A. CLASSIFICATION OF SUBJECT MATTER
*A61K31/7088*(2006.01), *A61K31/7105*(2006.01), *A61K31/711*(2006.01),
*A61K31/713*(2006.01), *A61K47/16*(2006.01), *A61K47/24*(2006.01), *A61K48/00*
(2006.01), *A61P35/00*(2006.01)
According to International Patent Classification (IPC) or to both national classification and IPC

B. FIELDS SEARCHED

Minimum documentation searched (classification system followed by classification symbols)
A61K31/7088, A61K31/7105, A61K31/711, A61K31/713, A61K47/16, A61K47/24,
A61K48/00, A61P35/00

Documentation searched other than minimum documentation to the extent that such documents are included in the fields searched
Jitsuyo Shinan Koho        1922-1996    Jitsuyo Shinan Toroku Koho   1996-2006
Kokai Jitsuyo Shinan Koho  1971-2006    Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 99/61032 A (Nippon Shinyaku Co., Ltd.),<br>02 December, 1999 (02.12.99),<br>Page 4, line 11 to page 6, line 21; page 11,<br>line 7 to page 16, line 3; pages 20 to 21<br>& EP 1086699 A1          & JP 2005-50492 A<br>& US 6545138 B1          & US 2003/091622 A1<br>& US 6746690 B2 | 8<br>1-7 |
| A | WO 99/48531 A (Nippon Shinyaku Co., Ltd.),<br>30 September, 1999 (30.09.99),<br>Page 16<br>& EP 1064950 A1          & JP 2005-37578 A<br>& EP 1064950 B1 | 1-8 |

| ☒ Further documents are listed in the continuation of Box C. | ☐ See patent family annex. |
|---|---|

| * Special categories of cited documents:<br>"A" document defining the general state of the art which is not considered    to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>27 June, 2006 (27.06.06) | Date of mailing of the international search report<br>04 July, 2006 (04.07.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/310647

C (Continuation).    DOCUMENTS CONSIDERED TO BE RELEVANT

| Category* | Citation of document, with indication, where appropriate, of the relevant passages | Relevant to claim No. |
|---|---|---|
| A | WO 99/20283 A  (Nippon Shinyaku Co., Ltd.),<br>29 April, 1999 (29.04.99),<br>Page 20<br>& EP 1029544 A1 | 1-8 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9920283 A
- WO 9948531 A
- WO 2004106511 A
- WO 9961032 A
- WO 9419314 A